(19) Europäisches Patentamt / European Patent Office / Office européen des brevets

(11) **EP 3 177 584 B1**

(12) **FASCICULE DE BREVET EUROPEEN**

(45) Date de publication et mention de la délivrance du brevet:
**10.10.2018 Bulletin 2018/41**

(21) Numéro de dépôt: **15744263.3**

(22) Date de dépôt: **04.08.2015**

(51) Int Cl.:
*C07C 7/13* (2006.01)      *C07C 15/08* (2006.01)
*B01J 20/18* (2006.01)     *B01J 20/28* (2006.01)

(86) Numéro de dépôt international:
**PCT/EP2015/067966**

(87) Numéro de publication internationale:
**WO 2016/020386 (11.02.2016 Gazette 2016/06)**

(54) **PROCÉDÉ DE SÉPARATION DU MÉTA-XYLÈNE UTILISANT UN ADSORBANT ZÉOLITHIQUE À HAUTE SURFACE EXTERNE**

VERFAHREN ZUR TRENNUNG VON META-XYLOL UNTER VERWENDUNG EINES ZEOLITHISCHEN ADSORPTIONSMITTELS MIT EINER GROSSEN EXTERNEN OBERFLÄCHE

METHOD FOR SEPARATING META-XYLENE USING A ZEOLITIC ADSORBENT WITH A LARGE EXTERNAL SURFACE AREA

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **05.08.2014 FR 1457623**

(43) Date de publication de la demande:
**14.06.2017 Bulletin 2017/24**

(73) Titulaires:
• **IFP Energies nouvelles**
**92500 Rueil-Malmaison (FR)**
• **ARKEMA FRANCE**
**92700 Colombes (FR)**

(72) Inventeurs:
• **LAROCHE, Catherine**
**F-69390 Vernaison (FR)**
• **LEFLAIVE, Philibert**
**F-69780 Mions (FR)**
• **BAUDOT, Arnaud**
**F-69390 Vernaison (FR)**
• **BOUVIER, Ludivine**
**F-64300 Orthez (FR)**
• **LUTZ, Cécile**
**F-64290 Gan (FR)**
• **NICOLAS, Serge**
**F-64000 Pau (FR)**

(74) Mandataire: **Casalonga**
**Casalonga & Partners**
**Bayerstraße 71/73**
**80335 München (DE)**

(56) Documents cités:
FR-A1- 2 988 718      FR-A1- 3 002 461
JP-A- H 069 438       US-A1- 2009 326 310
US-B1- 6 180 550

**Description**

L'invention concerne un procédé de séparation du *méta*-xylène de ses isomères présents dans un mélange gazeux ou liquide, mettant en oeuvre un adsorbant sous forme d'aggloméré comprenant de la zéolithe faujasite (FAU) de type NaY et/ou de type NaLiY et présentant une importante surface externe, ladite surface externe étant caractérisée par une population de mésopores déterminée par mesure d'adsorption d'azote. Le procédé de la présente invention permet l'obtention de *méta*-xylène de très haute pureté.

L'utilisation d'adsorbants zéolithiques contenant des zéolithes de type NaY et/ou NaLiY pour adsorber sélectivement le *méta*-xylène dans un mélange d'hydrocarbures aromatiques, est bien connue de l'art antérieur.

Par exemple, les brevets US4306107, US4326092, US5382747, US5900523 et US7728187 ainsi que FR2889698 et FR2889699 montrent que des adsorbants zéolithiques comprenant des aluminosilicates à base de sodium ou à base de sodium et de lithium, sont efficaces pour la séparation du *méta*-xylène présent dans des coupes aromatiques en C8 (coupes comprenant des hydrocarbures aromatiques à 8 atomes de carbone).

Les adsorbants décrits dans le brevet US5900523 sont utilisés comme agents d'adsorption dans les procédés en phase liquide, de préférence de type contre-courant simulé, similaires à ceux décrits dans le brevet US2985589 et qui s'appliquent, entre autres, aux coupes aromatiques en C8.

Dans les brevets listés ci-dessus, les adsorbants zéolithiques se présentent sous forme de cristaux à l'état de poudre ou sous forme d'agglomérés constitués majoritairement de poudre de zéolithe et jusqu'à 20% en poids de liant inerte.

La synthèse des zéolithes FAU s'effectue habituellement par nucléation et cristallisation de gels de silico-aluminates. Cette synthèse conduit à des cristaux (généralement sous forme de poudre) dont l'emploi à l'échelle industrielle est particulièrement malaisé (pertes de charges importantes lors des manipulations). On préfère alors les formes agglomérées de ces cristaux, sous forme de grains, de filés et autres agglomérés, ces dites formes pouvant être obtenues par extrusion, pastillage, atomisation et autres techniques d'agglomération connues de l'homme du métier. Ces agglomérés ne présentent pas les inconvénients inhérents aux matières pulvérulentes.

D'autre part, les cristaux de zéolithes sont le plus souvent préparés à partir de solutions aqueuses sodiques (par exemple solution aqueuse d'hydroxyde de sodium), et, si on le souhaite, les cations sodium peuvent être remplacés (échangés) en totalité ou en partie par d'autres cations, par exemple le lithium. Ces échanges cationiques peuvent être effectués avant et/ou après agglomération de la zéolithe pulvérulente avec le liant d'agglomération, selon des techniques classiques connues de l'homme du métier.

Les agglomérés, qu'ils soient sous forme de plaquettes, de billes, d'extrudés, et autres, sont en général constitués de cristaux de zéolithe(s), qui constituent l'élément actif (au sens de l'adsorption) et d'un liant d'agglomération. Ce liant d'agglomération est destiné à assurer la cohésion des cristaux entre eux dans la structure agglomérée, mais aussi doit permettre d'assurer une résistance mécanique suffisante auxdits agglomérés afin d'éviter, ou tout au moins de minimiser le plus possible, les risques de fractures, brisures ou cassures qui pourraient survenir lors de leurs utilisations industrielles pendant lesquelles les agglomérés sont soumis à de nombreuses contraintes, telles que vibrations, variations fortes et/ou fréquentes de pressions, mouvements et autres.

La préparation de ces agglomérés s'opère par exemple par empâtage de cristaux de zéolithe à l'état de poudre avec une pâte argileuse, dans des proportions de l'ordre de 80 à 90 % en poids de poudre de zéolithe pour 20% à 10% en poids de liant, puis mise en forme en billes, plaquettes ou extrudés, et traitement thermique à haute température pour cuisson de l'argile et réactivation de la zéolithe, le ou les échange(s) cationique(s), comme par exemple l'échange total ou partiel au lithium pouvant être effectué avant et/ou après l'agglomération de la zéolithe pulvérulente avec le liant.

On obtient des corps zéolithiques dont la granulométrie est de quelques millimètres, voire de l'ordre du millimètre, et qui, si le choix du liant d'agglomération et la granulation sont faits dans les règles de l'art, présentent un ensemble de propriétés satisfaisantes, en particulier de porosité, de résistance mécanique, de résistance à l'abrasion, et autres. Cependant, les propriétés d'adsorption de ces agglomérés sont évidemment réduites par rapport à la poudre active de départ en raison de la présence de liant d'agglomération inerte vis-à-vis de l'adsorption.

Divers moyens ont déjà été proposés pour pallier cet inconvénient du liant d'agglomération d'être inerte quant aux performances d'adsorption, parmi lesquels, la transformation de la totalité ou d'au moins une partie du liant d'agglomération en zéolithe active du point de vue de l'adsorption. Cette opération est maintenant bien connue de l'homme du métier, par exemple sous la dénomination de « zéolithisation ». Pour effectuer cette opération, on utilise des liants zéolithisables, le plus souvent appartenant à la famille de la kaolinite, et de préférence préalablement calcinés à des températures généralement comprises entre 500°C et 700°C.

Le document US4306107 décrit l'utilisation, en tant qu'adsorbant sélectif du *méta*-xylène, d'une zéolithe Y, dans laquelle les sites cationiques échangeables sont occupés par des atomes de sodium. Pour obtenir une sélectivité satisfaisante en faveur du *méta*-xylène, il est recommandé d'utiliser une zéolithe partiellement hydratée, présentant une perte au feu à 950°C de 2% à 7% en poids du poids initial de l'adsorbant. Il est préconisé dans ce document une séparation par un procédé en lit mobile simulé à une température comprise entre 20°C et 250°C et à une pression comprise entre la pression atmosphérique et 3,5 MPa (35 bars), valeur choisie de manière à maintenir la charge sous forme liquide. Le désorbant choisi est le toluène. L'occupation des sites échangeables de la zéolithe par des ions sodium et l'activation afin d'obtenir la perte au feu souhaitée permettent d'obtenir des agglomérés qui présentent, du point de vue de l'adsorption du *méta*-xylène contenu dans les coupes aromatiques en C8, des propriétés améliorées de capacité et de sélectivité.

Outre une capacité d'adsorption élevée et de bonnes propriétés de sélectivité vis-à-vis de l'espèce à séparer du mélange réactionnel, l'adsorbant doit présenter de bonnes propriétés de transfert de matière afin de garantir un nombre de plateaux théoriques suffisants pour réaliser une séparation efficace des espèces en mélange, comme l'indique Ruthven dans l'ouvrage intitulé « Principles of Adsorption and Adsorption Processes » (« Principes de l'Adsorption et des Procédés d'Adsorption »), John Wiley & Sons, (1984), pages 326 et 407. Ruthven indique (ibid., page 243), que, dans le cas d'un adsorbant aggloméré, le transfert de matière global dépend de l'addition de la résistance diffusionnelle intra-cristalline et de la résistance diffusionnelle entre les cristaux.

La résistance diffusionnelle intra-cristalline est proportionnelle au carré des diamètres des cristaux et inversement proportionnelle à la diffusivité intra-cristalline des molécules à séparer.

La résistance diffusionnelle entre les cristaux (également appelée « résistance macroporeuse ») est quant à elle proportionnelle au carré des diamètres des agglomérés, inversement proportionnelle à la porosité contenue dans les macropores et mésopores (c'est-à-dire les pores dont le diamètre est supérieur à 2 nm) au sein de l'aggloméré, et inversement proportionnelle à la diffusivité des molécules à séparer dans cette porosité.

La taille des adsorbants agglomérés est un paramètre important lors de l'utilisation de l'adsorbant dans l'application industrielle, car elle détermine la perte de charge au sein de l'unité industrielle et l'uniformité du remplissage. La distribution granulométrique des agglomérés doit donc être étroite, et centrée sur des diamètres moyens en nombre compris typiquement entre 0,40 mm et 0,65 mm afin d'éviter des pertes de charges excessives. La porosité contenue dans les macropores et mésopores ne participe pas à la capacité d'adsorption. Par conséquent, l'homme du métier ne cherchera pas à l'augmenter dans le but de réduire la résistance diffusionnelle macroporeuse, sachant que cela se ferait au détriment de la capacité d'adsorption volumique.

Pour estimer l'amélioration de la cinétique de transfert, il est possible d'utiliser la théorie des plateaux décrite par Ruthven dans « *Principles of Adsorption and Adsorption Processes* », *ibid.,* pages 248-250. Cette approche est basée sur la représentation d'une colonne par un nombre fini de réacteurs hypothétiques idéalement agités (étages théoriques). La hauteur équivalente de plateaux théoriques est une mesure directe de la dispersion axiale et de la résistance au transfert de matière du système.

Pour une structure zéolithique donnée, une taille d'adsorbant donnée et une température de fonctionnement donnée, les diffusivités sont fixées, et un des moyens d'améliorer le transfert de matière consiste à réduire le diamètre des cristaux. Un gain sur le transfert global sera ainsi obtenu en réduisant la taille des cristaux.

L'homme du métier va donc chercher à réduire le plus possible le diamètre des cristaux de zéolithes afin d'améliorer le transfert de matière.

Le brevet US7728187 revendique ainsi un procédé de séparation du méta-xylène d'un mélange d'hydrocarbures aromatiques en C8 consistant en la mise en contact du mélange, dans des conditions d'adsorption, avec adsorbant, dans lesquels les cristaux de zéolithe Y échangée au sodium sont de taille comprise entre 0,05 $\mu$m et 0,7 $\mu$m et ceci afin d'améliorer les performances en transfert de matière.

La demanderesse a néanmoins observé que la synthèse, la filtration, la manipulation et l'agglomération de cristaux de zéolithe dont la taille est inférieure à 0,7 $\mu$m mettent en oeuvre des procédés lourds, peu économiques et donc difficilement industrialisables. De plus, de tels adsorbants comportant des cristaux de taille inférieure à 0,7 $\mu$m, s'avèrent également plus fragiles, et il devient alors nécessaire d'augmenter le taux de liant d'agglomération afin de renforcer la cohésion des cristaux entre eux au sein de l'aggloméré.

Toutefois, l'augmentation du taux de liant d'agglomération conduit à une densification des agglomérés, à l'origine d'une augmentation de la résistance diffusionnelle macroporeuse. Ainsi, malgré une résistance diffusionnelle intra-cristalline réduite du fait de la diminution de la taille des cristaux, l'augmentation de la résistance diffusionnelle macroporeuse en raison de la densification de l'aggloméré, ne permet pas une amélioration du transfert de matière global. Dans le cadre de la présente invention, le terme « aggloméré » peut être employé en lieu et place du terme « adsorbant » et doit être compris comme définissant le même objet.

Il reste par conséquent un besoin pour des matériaux adsorbants zéolithiques préparés à partir de zéolithe de type FAU facilement manipulables au niveau industriel, c'est-à-dire dont les cristaux constitutifs sont avantageusement de taille supérieure à 0,7 $\mu$m, mais présentant un transfert de matière global amélioré par rapport à un adsorbant préparé à partir de cristaux de zéolithe de type FAU conventionnels de taille identique (c'est-à-dire supérieure à 0,7 $\mu$m), tout en conservant une capacité d'adsorption élevée.

De tels matériaux adsorbants zéolithiques à base de zéolithe de type FAU, et en particulier contenant des zéolithes de type NaY et/ou NaLiY pourraient apporter des améliorations significatives dans les procédés de séparation des xylènes présents sous forme de mélanges d'isomères dans les coupes d'hydrocarbures aromatiques en C8, et en particulier pourraient grandement améliorer l'adsorption sélective de *méta*-xylène dans les mélanges d'hydrocarbures aromatiques en C8.

La présente invention a ainsi pour premier objet de proposer un procédé de séparation du *méta*-xylène des coupes aromatiques en C8, en phase gaz ou en phase liquide, mettant en oeuvre un adsorbant zéolithique sous forme d'aggloméré(s) aux propriétés optimisées. L'adsorbant zéolithique mis en oeuvre dans le procédé selon l'invention présente notamment des propriétés maximales de sélectivité vis-à-vis du *méta*-xylène et de transfert de matière, tout en présentant une résistance mécanique et une capacité d'adsorption élevées. Le procédé selon l'invention est de préférence un procédé de séparation du *méta*-xylène en phase liquide, de préférence un procédé de séparation du *méta*-xylène en phase liquide de type contre-courant simulé.

Plus précisément, la présente invention concerne un procédé de séparation du *méta*-xylène mettant en oeuvre un adsorbant zéolithique comprenant au moins une zéolithe faujasite de type Y comprenant du sodium ou du sodium et du lithium, procédé dans lequel la surface externe dudit adsorbant zéolithique, mesurée par adsorption d'azote, est supérieure à 40 m$^2$.g$^{-1}$, de préférence supérieure à 50 m$^2$.g$^{-1}$, et de préférence encore comprise entre 60 m$^2$.g$^{-1}$ et 200 m$^2$.g$^{-1}$, et plus préférentiellement comprise entre 80 m$^2$.g$^{-1}$ et 200 m$^2$.g$^{-1}$, et de manière encore plus préférée entre 100 m$^2$.g$^{-1}$ et 200 m$^2$.g$^{-1}$ ladite surface externe étant associée à une population de mésopores de diamètre moyen compris entre 2 nm et 50 nm. Le terme « associée » dans la phrase précédente indique que la population de mésopores contribue à la valeur mesurée de la surface externe, en plus de la surface externe des cristaux de zéolithe(s).

Le diamètre moyen est déterminé par la méthode Barrett-Joyner-Halenda (méthode BJH, E. P.Barrett, L. G. Joyner, P. P. Halenda, "The Détermination of Pore Volume and Area Distributions in Porous Substances. I. Computations form Nitrogen Isotherms", J. Am. Chem. Soc., 73(1), (1951) 373-380), à partir de l'isotherme d'adsorption d'azote à 77K. Avantageusement, la distribution en volume de diamètre moyen ainsi déterminé, représentée graphiquement par $d$V/$d$Dm ou $d$V/$d$logDm en fonction du diamètre moyen Dm, correspond à une distribution unimodale et étroite.

Dans ce qui va suivre, et à moins d'une autre indication, les bornes d'un domaine de valeurs sont comprises dans ce domaine, notamment dans les expressions « compris entre » et « allant de ... à ... ».

Par « distribution unimodale », on entend une distribution ne présentant qu'un seul pic. Une distribution unimodale de diamètre moyen est ainsi caractérisée par un seul pic, pour lequel la valeur du diamètre moyen au sommet du pic est appelée « mode », ou encore valeur dominante, et représente la valeur la plus fréquente de la distribution. De manière préférée, la population de mésopores dudit adsorbant zéolithique présente des diamètres moyens au mode compris entre 2 nm et 30 nm et de préférence compris entre 2 nm et 20 nm. Lorsqu'une distribution présente deux pics séparés par un creux, on dit que la distribution est bimodale. L'invention ne concerne pas le cas de distribution bimodale voire multimodale, c'est-à-dire de distribution où il existe plusieurs zones de concentration des valeurs séparées par des discontinuités. De telles distributions sont caractéristiques de la présence de plusieurs populations de pores de diamètres moyens distincts.

Le terme « étroite », utilisé pour caractériser la distribution de diamètre moyen des mésopores, indique que la largeur à mi-hauteur de la distribution autour du mode est inférieure à 20 nm, de préférence inférieure à 15 nm, de manière préférée comprise entre 10 nm et 0,1 nm et de manière encore préférée comprise entre 5 nm et 0,5 nm, comme décrit plus loin dans les techniques de caractérisation.

Le procédé selon l'invention met de préférence en oeuvre un adsorbant zéolithique comprenant de préférence au moins une phase non zéolithique dont au moins une partie est une argile ou un mélange d'argiles utilisée(s) dans le mode de préparation comme liant d'agglomération permettant la cohésion entre eux des cristaux, issus de la synthèse, avant d'être optionnellement tout ou partiellement zéolithisée, c'est-à-dire transformée en zéolithe active pour l'adsorption des molécules considérées, c'est-à-dire préférentiellement convertie en zéolithe, de préférence zéolithe FAU, de préférence encore de type Y.

Selon un mode de réalisation de l'invention, l'adsorbant zéolithique peut présenter une teneur en lithium inférieure à 8%, de préférence comprise entre 0 et 4%, exprimée en poids d'oxyde de lithium Li$_2$O par rapport à la masse totale de l'adsorbant.

Selon un autre mode de réalisation de l'invention la teneur totale en ions alcalins ou alcalino-terreux autres que le lithium et le sodium est comprise entre 0 et 1%, cette teneur totale étant exprimée respectivement en masse d'oxyde de lithium Li$_2$O et d'oxyde de sodium Na$_2$O, par rapport à la masse totale de l'adsorbant zéolithique.

De préférence l'adsorbant zéolithique selon la présente invention est un adsorbant zéolithique comprenant au moins une zéolithe faujasite de type Y, autrement dit un adsorbant à base de zéolithe(s) FAU, généralement référencée(s) sous le nom de zéolithe FAU de type Y. Par « zéolithe FAU de type Y », on entend une zéolithe dont le rapport atomique Si/Al est supérieur à 1,50, de préférence compris entre 1,60 et 6,00 , de préférence entre 1,80 et 4,00, et de manière encore plus préférée entre 2,00 et 2,80.

Selon un mode de réalisation préféré, le procédé selon l'invention utilise un adsorbant zéolithique comprenant des cristaux mésoporeux de zéolithe Y. Par « mésoporeux », on entend des cristaux zéolithiques qui présentent, conjointement à la microporosité inhérente à la structure de la zéolithe, des cavités internes de taille nanométrique (mésoporosité), facilement identifiables par observation au moyen d'un microscope électronique à transmission (MET ou « TEM » en langue anglaise), comme décrit par exemple dans US7785563.

Selon un autre mode de réalisation, aucune structure zéolithique autre que la structure FAU n'est détectée par diffraction des rayons X (connue de l'homme du métier sous l'acronyme DRX) dans l'adsorbant zéolithique utilisé dans le procédé de la présente invention. Par « aucune », on entend moins de 5% en masse, de préférence moins de 2% en masse, de zéolithe de structure autre que structure FAU, par rapport à la masse totale de l'adsorbant, les fractions massiques étant déterminées par DRX.

Selon encore un autre mode de réalisation préféré, le procédé selon l'invention utilise un adsorbant zéolithique dont la fraction massique de zéolithe(s) FAU de type Y est supérieure ou égale à 80% par rapport au poids total de l'adsorbant, le complément à 100% étant de préférence constitué de phase non zéolithique.

Le procédé selon l'invention utilise un adsorbant pouvant contenir une phase non zéolithique, c'est-à-dire une phase non cristalline qui est essentiellement inerte vis-à-vis de l'adsorption. La fraction massique de zéolithe(s) (taux de cristallinité de l'adsorbant) peut être déterminée par analyse par diffraction de rayons X, connue de l'homme du métier sous l'acronyme DRX, comme indiqué précédemment.

Selon encore un autre mode de réalisation préféré, le procédé selon l'invention utilise un adsorbant zéolithique présentant un rapport atomique Si/Al compris entre 1,50 et 6,50, de préférence entre 2,00 et 6,00, de préférence encore entre 2,00 et 4,00, et de manière encore plus préférée entre 2,20 et 2,80.

Selon un mode de réalisation préféré, le procédé selon l'invention utilise un adsorbant zéolithique qui présente une perte au feu mesurée à 950°C selon la norme NF EN 196-2 inférieure ou égale à 7%, de préférence comprise entre 0 et 6%, de manière préférée entre 0 et 4%, de manière encore préférée entre 0 et 3% et avantageusement entre 0 et 2,5%.

Le procédé selon l'invention utilise de préférence un adsorbant zéolithique comprenant à la fois des macropores, des mésopores et des micropores. Par « macropores », on entend des pores dont le diamètre est supérieur à 50 nm, de préférence compris entre 50 nm et 400 nm. Par « mésopores », on entend des pores dont le diamètre est compris entre 2 nm et 50 nm, bornes non incluses. Par « micropores », on entend des pores dont le diamètre est inférieur à 2 nm.

Selon encore un autre mode de réalisation préféré, l'adsorbant zéolithique de la présente invention présente un volume total contenu dans les macropores et les mésopores (somme du volume macroporeux et du volume mésoporeux) mesuré par intrusion de mercure, avantageusement compris entre 0,20 $cm^3.g^{-1}$ et 0,70 $cm^3.g^{-1}$, de préférence compris entre 0,20 $cm^3.g^{-1}$ et 0,55 $cm^3.g^{-1}$ et de manière très préférée compris entre 0,20 $cm^3.g^{-1}$ et 0,50 $cm^3.g^{-1}$.

Le ratio (volume macroporeux)/(volume macroporeux + volume mésoporeux) est de préférence compris entre 0,2 et 1, de manière très préférée compris entre 0,4 et 0,8, et de manière encore plus préférée entre 0,45 et 0,65.

On préfère également dans le cadre de la présente invention un procédé mettant en oeuvre un adsorbant zéolithique dont le volume microporeux évalué par la méthode de t-plot à partir de l'isotherme d'adsorption d'azote ($N_2$) à une température de 77 K, est compris entre 0,145 $cm^3.g^{-1}$ et 0,350 $cm^3.g^{-1}$, mieux entre 0,155 $cm^3.g^{-1}$ et 0,350 $cm^3.g^{-1}$, de préférence compris entre 0,165 $cm^3.g^{-1}$ et 0,350 $cm^3.g^{-1}$, mieux encore entre 0,175 $cm^3.g^{-1}$ et 0,350 $cm^3.g^{-1}$, et de préférence encore compris entre 0,205 $cm^3.g^{-1}$ et 0,320 $cm^3.g^{-1}$. Ladite mesure de volume microporeux est calculée après dégazage sous vide (P < $6,7.10^{-4}$ Pa), à une température comprise entre 300°C et 450°C pendant une durée allant de 9 heures à 16 heures, par exemple à 400°C pendant 10 heures.

Le procédé selon l'invention met de préférence en oeuvre un adsorbant zéolithique sous la forme d'agglomérés, c'est-à-dire qu'il comprend des cristaux mésoporeux d'au moins une zéolithe Y telle que définie précédemment, lesdits cristaux présentant un diamètre moyen en nombre compris entre 0,1 $\mu m$ et 20 $\mu m$, de préférence entre 0,5 $\mu m$ et 20 $\mu m$, de préférence encore entre 0,7 $\mu m$ et 10 $\mu m$, et plus préférentiellement encore entre 0,8 $\mu m$ et 10 $\mu m$, et plus préférentiellement encore entre 1 $\mu m$ et 5 $\mu m$.

Il a en effet été constaté par la demanderesse que les adsorbants zéolithiques selon l'invention préparés à partir de cristaux de haute surface externe associée à une (1) population de mésopores présentent un transfert de matière global amélioré par rapport à des adsorbants préparés à partir de cristaux de zéolithe(s) de type Y conventionnels, y compris lorsque lesdits cristaux sont de taille supérieure aux cristaux conventionnels.

La présente invention permet donc la mise à disposition d'un procédé de séparation du *méta*-xylène amélioré par rapport à l'art antérieur tout en remédiant aux problèmes liés à la filtration, la manipulation et l'agglomération de poudres zéolithiques sub-microniques lors du procédé de fabrication de l'adsorbant.

La surface externe des cristaux contenu dans l'adsorbant du procédé décrit ci-dessus est calculée par la méthode du t-plot à partir de l'isotherme d'adsorption d'azote à une température de 77K, après dégazage sous vide (P < $6,7.10^{-4}$ Pa), à une température comprise entre 300°C et 450°C pendant une durée allant de 9 heures à 16 heures, de préférence à 400°C pendant 10 heures.

Les cristaux de zéolithe Y présentant une importante surface externe peuvent être obtenus selon diverses méthodes connues de l'homme du métier et par exemple selon la synthèse décrite dans CN103214003.

Il est également possible de préparer lesdits cristaux par synthèse par ensemencement et/ou par ajustement des conditions opératoires de synthèse tels que le rapport $SiO_2/Al_2O_3$, la teneur en sodium et l'alcalinité du mélange de synthèse ou encore selon des procédés de post-traitement de cristaux de zéolithe Y conventionnels et connus de l'homme du métier.

Les procédés de post-traitements consistent généralement à éliminer des atomes du réseau zéolithique déjà formé, soit par un ou plusieurs traitements acides qui désaluminent le solide, traitement(s) suivi(s) par un ou plusieurs lavage(s) à la soude afin d'éliminer les résidus aluminiques formés, comme décrit par exemple par D. Verboekend et coll., dans Adv. Funct. Mater., 22, (2012), 916-928, soit encore par des traitements qui associent l'action d'un acide et celle d'un agent structurant qui améliorent l'efficacité du traitement acide, comme décrit par exemple dans la demande WO2013/106816.

Les procédés de synthèse directe de ces zéolithes (c'est-à-dire des procédés de synthèse autre que le post-traitement) sont préférés et font généralement intervenir un ou plusieurs agents structurants ou gabarits sacrificiels. Ainsi, et selon encore un autre objet, la présente invention concerne un procédé de séparation des isomères comme défini précédemment, dans lequel les cristaux de zéolithe(s) de l'adsorbant zéolithique sont préparés par synthèse directe mettant en oeuvre un ou plusieurs agents structurants ou gabarits sacrificiels.

Les gabarits sacrificiels pouvant être utilisés peuvent être de tout type connu de l'homme du métier et notamment ceux décrits dans la demande WO2007/043731. Selon un mode de réalisation préféré, le gabarit sacrificiel est avantageusement choisi parmi les organosilanes et plus préférentiellement parmi le chlorure de [3-(triméthoxysilyl)propyl]-octadécyldiméthylammonium, le chlorure de [3-(triméthoxysilyl)propyl]hexadécyldiméthyl-ammonium, le chlorure de [3-(triméthoxysilyl)propyl]dodécyldiméthylammonium, le chlorure de [3-(triméthoxysilyl)propyl]octylammonium, la N-[3-(triméthoxysilyl)-propyl]-aniline, le 3-[2-(2-amino-éthylamino)éthylamino]propyltriméthoxysilane, la N-[3-(trimétho-xysilyl)propyl]-N'-(4-vinylbenzyl)éthylènediamine, le triéthoxy-3-(2-imidazolin-1-yl)propyl-silane, la 1-[3-(triméthoxysilyl)propyl]urée, la N-[3-(triméthoxysilyl)propyl]-éthylènediamine, le [3-(diéthylamino)propyl]triméthoxysilane, le (3-glycidyloxypropyl)triméthoxysilane, le méthacrylate de 3-(triméthoxysilyl)propyle, le [2-(cyclohexényl)éthyl]triéthoxysilane, le dodécyltriéthoxysilane, l'hexadécyltriméthoxysilane, le (3-aminopropyl)triméthoxysilane, le (3-mercaptopropyl)triméthoxysilane, le (3-chloropropyl)triméthoxysilane, ainsi que les mélanges de deux ou plusieurs d'entre eux en toutes proportions.

Parmi les gabarits sacrificiels listés ci-dessus, on préfère tout particulièrement le chlorure de [3-(triméthoxysilyl)propyl]octadécyldiméthylammonium, ou TPOAC.

On peut également utiliser des gabarits sacrificiels de masse molaire plus élevée et par exemple les PPDA (Polymer Poly-DiallyldimethylAmmonium), PVB (PolyVinyl Butyral) et autres composés oligomères connus dans le domaine pour augmenter le diamètre des mésopores.

La taille des cristaux de zéolithe Y de l'adsorbant du procédé selon l'invention est mesurée par observation au microscope électronique à balayage (MEB). Comme indiqué précédemment, de manière préférée, le diamètre moyen en nombre des cristaux est compris entre 0,1 $\mu$m et 20 $\mu$m, de préférence entre 0,5 $\mu$m et 20 $\mu$m, de préférence encore entre 0,7 $\mu$m et 10 $\mu$m, et plus préférentiellement encore entre 0,8 $\mu$m et 10 $\mu$m, et plus préférentiellement encore entre 1 $\mu$m et 5 $\mu$m.

Cette observation MEB permet également de confirmer la présence de phase non zéolithique comprenant par exemple du liant résiduel (non converti lors de l'étape de zéolithisation) ou toute autre phase amorphe dans les adsorbants.

Dans le présent document, on emploie l'appellation « diamètre moyen en nombre » ou bien « taille », notamment pour les cristaux de zéolithe. La méthode de mesure de ces grandeurs est explicitée plus loin dans la description.

Les proportions de liant d'agglomération (voir définition plus loin) et de zéolithe mises en oeuvre dans les adsorbant du procédé selon l'invention sont typiquement celles de l'art antérieur, c'est-à-dire de 5 parties à 20 parties en poids de liant pour 95 parties à 80 parties en poids de zéolithe. L'adsorbant qu'il soit sous forme de billes, d'extrudés ou autres, a de préférence un diamètre moyen en volume, ou une longueur (plus grande dimension lorsqu'il n'est pas sphérique), comprise entre 0,2 mm et 2 mm, et en particulier comprise entre 0,2 mm et 0,8 mm et de préférence entre 0,4 mm et 0,65 mm.

Le liant compris dans le matériau aggloméré zéolithique du procédé de la présente invention comprend, et de préférence consiste en, une argile ou un mélange d'argiles. Ces argiles sont de préférence choisies parmi les kaolins, kaolinites, nacrites, dickites, halloysites, attapulgites, sépiolites, montmorillonites, bentonites, illites et métakaolins, ainsi que les mélanges de deux ou plusieurs d'entre elles en toutes proportions. Le kaolin est préféré et le plus couramment utilisé.

Parmi les additifs éventuellement mis en oeuvre lors de la fabrication de l'adsorbant du procédé selon l'invention, on peut trouver une source de silice de tout type connu de l'homme du métier, spécialiste de la synthèse de zéolithes, par exemple de la silice colloïdale, des diatomées, de la perlite, des cendres de calcination (« fly ash » en langue anglaise), du sable, ou toute autre forme de silice solide.

Dans le cadre de la présente invention, la résistance mécanique est mesurée par la méthode Shell série SMS1471-74 adaptée pour des agglomérés de taille inférieure à 1,6 mm. Cette résistance mécanique, mesurée pour l'adsorbant zéolithique défini précédemment, est généralement comprise entre 1,5 MPa et 4 MPa, de préférence entre 1,7 MPa et 4 MPa de préférence encore entre 1,8 MPa et 4 MPa et de manière tout à fait préférée entre 2 MPa et 4 MPa.

Lors de la fabrication de l'adsorbant du procédé selon l'invention, outre les cristaux de zéolithe Y et le liant, d'autres additifs peuvent également être ajoutés, par exemple des additifs destinés à faciliter l'agglomération ou à améliorer le durcissement des agglomérés formés tels que la lignine, l'amidon, la carboxyméthylcellulose, et autres additifs connus de l'homme du métier.

Lors de la fabrication de l'adsorbant du procédé selon l'invention, la zéolithisation du liant d'agglomération peut être pratiquée selon toute méthode maintenant bien connue de l'homme du métier et peut par exemple être réalisée par immersion du produit issu de l'étape d'agglomération dans une solution basique alcaline, en général aqueuse, par exemple une solution aqueuse d'hydroxyde de sodium et/ou d'hydroxyde de potassium.

En règle générale, la concentration de la solution alcaline de zéolithisation est de préférence comprise entre 0,5 M et 5 M. La zéolithisation s'opère de préférence à chaud, à une température supérieure à la température ambiante, et typiquement à des températures de l'ordre de 80°C à 100°C, par exemple comprises entre la température ambiante (soit environ 20°C) et la température d'ébullition de la solution alcaline de zéolithisation. La durée du processus de zéolithisation est généralement comprise entre quelques dizaines de minutes et quelques heures, de préférence entre environ 1 heure et 8 heures.

L'étape d'échange cationique éventuelle des ions sodium par des ions lithium peut s'effectuer selon les méthodes classiques connues de l'homme du métier, et le plus souvent par mise en contact des agglomérés issus de l'étape d'agglomération avec un sel de lithium, tel que le chlorure de lithium (LiCl), en solution aqueuse à une température comprise entre la température ambiante et 100°C, et de préférence comprise entre 80°C et 100°C.

Comme indiqué précédemment, lorsque l'adsorbant comprend une zéolithe NaLiY, il est également possible d'agglomérer des cristaux de zéolithe Y contenant déjà des ions sodium et lithium (pré-échange partiel des cations présents dans la zéolithe de type Y de départ, typiquement cations sodium, par des ions lithium).

De manière surprenante, la demanderesse a observé que l'étape d'échange cationique, qui peut-être délicate en raison de la relative fragilité de la structure des cristaux zéolithiques, n'affecte pas les propriétés intrinsèques de surface externe et du volume microporeux (ramené à la masse de l'adsorbant une fois échangé) desdits cristaux zéolithiques.

Après la ou les étape(s) d'échange(s) cationique(s), on procède ensuite à un lavage, généralement et de préférence à l'eau, puis d'un séchage de l'aggloméré ainsi obtenu.

L'activation qui suit le séchage, est conduite de manière classique, selon les méthodes connues de l'homme du métier, par exemple à une température en général comprise entre 100°C et 400°C, de préférence entre 200°C et 300°C pendant une durée déterminée en fonction de la teneur en eau et de la perte au feu souhaitées, typiquement de 1 à 6 heures.

L'invention concerne un procédé de séparation de *méta*-xylène à partir de coupes d'isomères aromatiques à 8 atomes de carbone, utilisant, comme agent d'adsorption du *méta*-xylène, un adsorbant zéolithique comprenant au moins une zéolithe FAU de type Y telle que définie précédemment, ledit procédé étant opéré en phase liquide, ou en phase gazeuse.

On peut ainsi séparer le produit désiré (*méta*-xylène) par chromatographie liquide d'adsorption préparative (en batch), et avantageusement en continu en lit mobile simulé, c'est-à-dire à contre-courant simulé ou à co-courant simulé, et plus particulièrement à contre-courant simulé.

Les conditions opératoires d'une unité industrielle d'adsorption de type contre-courant simulé sont en général les suivantes :

- nombre de lits : 6 à 30,
- nombre de zones : au moins 4 zones de fonctionnement, chacune étant localisées entre un point d'alimentation et un point de soutirage,
- température comprise entre 100°C et 250°C, de préférence entre 120°C et 190°C,
- pression de l'unité industrielle comprise entre la pression de bulle des xylènes à la température du procédé et 3 MPa,
- rapport des débits désorbant/charge compris entre 0,7 et 6,0,
- taux de recyclage compris entre 2,0 et 20, de préférence entre 2,5 et 10.

On pourra sur ce sujet se référer à l'enseignement des brevets FR2889698 et FR2889699.

Le solvant de désorption peut être tout désorbant connu de l'homme du métier et dont le point d'ébullition est inférieur à celui de la charge, tel que le toluène ou l'indane mais aussi un désorbant dont le point d'ébullition est supérieur à celui de la charge, tel que la tétraline. La sélectivité des adsorbants utilisé dans le procédé selon l'invention pour l'adsorption du *méta*-xylène contenu dans des coupes aromatiques en C8 est optimale lorsque leur perte au feu mesurée à 950°C est de préférence inférieure ou égale à 7%, de préférence comprise entre 0 et 6,0%, de manière très préférée entre 0 et 4,0%, de manière plus préférée entre 0 et 3,0%.

Le procédé de séparation du *méta*-xylène selon la présente invention utilise des adsorbants zéolithiques agglomérés possédant à la fois les caractéristiques des adsorbants zéolithiques conventionnels connus dans les procédés de séparation du *méta*-xylène de l'art antérieur, notamment les propriétés mécaniques et de microporosité, les caractéristiques de transfert de matière global étant maximisés par rapport à des adsorbants zéolithiques à base de cristaux conventionnels.

Les exemples qui suivent permettent d'illustrer l'objet de l'invention, et sont fournis à titre indicatif seulement, sans toutefois être destinés en aucune façon à limiter les divers modes de réalisation de la présente invention.

Dans les exemples qui suivent, les propriétés physiques des agglomérés sont évaluées par les méthodes connues de l'homme du métier, dont les principales d'entre elles sont rappelées ci-dessous :

TECHNIQUES DE CARACTERISATION

**Granulométrie des cristaux zéolithiques** - **Détection des mésopores**

L'estimation du diamètre moyen en nombre des cristaux de zéolithe de type Y contenus dans les adsorbants utilisés dans le procédé selon l'invention est réalisée par observation au microscope électronique à balayage (MEB).

Afin d'estimer la taille des cristaux de zéolithe sur les échantillons, on effectue un ensemble de clichés à un grossissement d'au moins 5000. On mesure ensuite le diamètre d'au moins 200 cristaux à l'aide d'un logiciel dédié. La précision est de l'ordre de 3%.

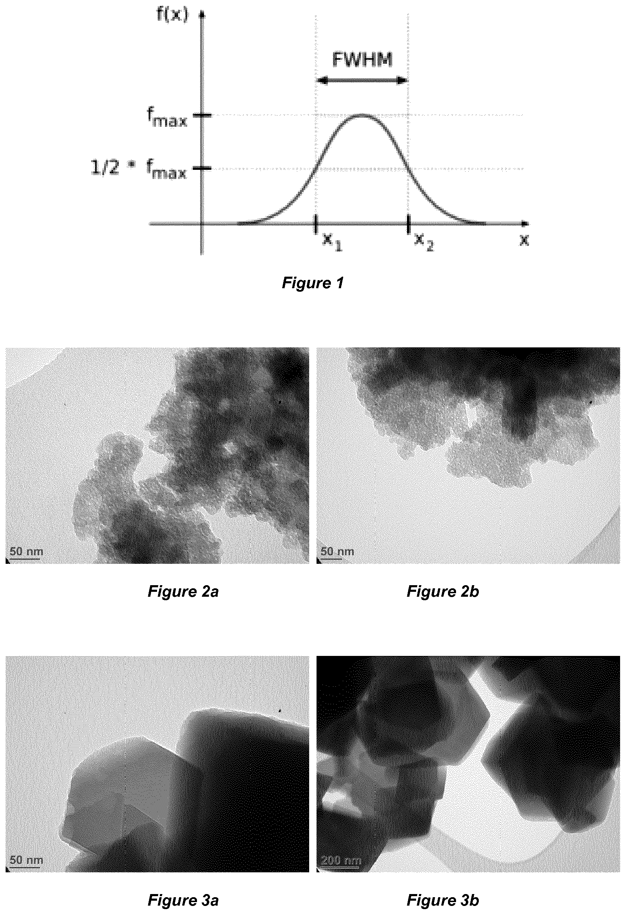

Comme indiqué dans US7785563, le MET permet en outre de vérifier si les cristaux de zéolithe(s) contenus dans l'adsorbant sont des cristaux de zéolithe pleins (i.e. non mésoporeux) ou des agrégats de cristaux de zéolithes pleins ou des cristaux mésoporeux (cf. la comparaison des clichés MET de la Figure 2a et de la Figure 2b, où la mésoporosité est clairement visible et la Figure 3a et la Figure 3b b qui montrent des cristaux pleins). L'observation MET permet ainsi de visualiser la présence ou l'absence des mésopores). De manière préférée, les adsorbants du procédé selon l'invention contiennent très majoritairement, i.e. typiquement plus de 80% et de préférence plus de 90% en nombre des cristaux zéolithiques mésoporeux et non des cristaux pleins. Cette analyse statistique est effectuée avantageusement par analyse d'au moins 50 clichés MET ou MEB (MEB sur sections d'échantillons obtenues par polissage ionique).

**Analyse chimique des adsorbants zéolithiques**

**Rapport Si/Al et taux d'échange**

Une analyse chimique élémentaire de l'adsorbant zéolithique du procédé selon l'invention peut être réalisée par différentes techniques analytiques connues par l'homme de l'art. Parmi ces techniques, on peut citer la technique d'analyse chimique par fluorescence de rayons X telle que décrite dans la norme NF EN ISO 12677 sur un spectromètre dispersif en longueur d'onde (WDXRF) du type par exemple Tiger S8 de BRUKER ou par des méthodes alternatives classiques telle que les méthodes par spectrométrie d'absorption atomique (AAS) et spectrométrie d'émission atomique avec plasma induit par haute fréquence (ICP-AES) décrites dans les normes NF EN ISO 21587-3 ou NF EN ISO 21079-3 sur un appareil de type par exemple Perkin Elmer 4300DV.

Le spectre de fluorescence X a l'avantage de dépendre très peu de la combinaison chimique de l'élément, ce qui offre une détermination précise, à la fois quantitative et qualitative. On obtient de manière classique après étalonnage pour chaque oxyde $SiO_2$ et $Al_2O_3$, ainsi que l'oxyde de sodium, une incertitude de mesure inférieure à 0,4% en poids. La méthode ICP-AES est particulièrement adaptée pour mesurer la teneur en lithium qui permet de calculer la teneur en oxyde de lithium.

Ces analyses chimiques élémentaires permettent à la fois de vérifier le rapport atomique Si/Al de la zéolithe utilisée lors de la préparation de l'adsorbant, ainsi que le rapport atomique Si/Al de l'adsorbant, et de vérifier la qualité de l'échange ionique. Dans la description de la présente invention, l'incertitude de mesure du rapport atomique Si/Al est de $\pm$ 5%.

Le taux d'échange des ions sodium par les ions lithium est estimé en évaluant le rapport entre le nombre de moles d'oxyde de lithium, $Li_2O$, et le nombre de moles de l'ensemble ($Li_2O$ + $Na_2O$). Il est à noter que les teneurs en différents oxydes sont donnés, en pourcentage en poids par rapport au poids total de l'adsorbant zéolithique anhydre.

**Granulométrie des adsorbants zéolithiques**

La détermination du diamètre moyen en volume des adsorbants zéolithiques du procédé selon l'invention est effectuée par analyse de la distribution granulométrique d'un échantillon d'aggloméré par imagerie selon la norme ISO 13322-2:2006, en utilisant un tapis roulant permettant à l'échantillon de passer devant l'objectif de la caméra.

Le diamètre moyen en volume est ensuite calculé à partir de la distribution granulométrique en appliquant la norme ISO 9276-2:2001. Dans le présent document, on emploie l'appellation « diamètre moyen en volume » ou bien « taille » pour les agglomérés zéolithiques. La précision est de l'ordre de 0,01 mm pour la gamme de taille d'agglomérés du procédé selon l'invention.

**Résistance mécanique des adsorbants zéolithiques**

La résistance à l'écrasement d'un lit d'adsorbants zéolithiques tels que décrits dans la présente invention est caractérisée selon la méthode Shell série SMS1471-74 (Shell Method Series SMS1471-74 « Détermination of Bulk Crushing Strength of Catalysts. Compression-Sieve Method »), associée à l'appareil « BCS Tester » commercialisé par la société Vinci Technologies. Cette méthode, initialement destinée à la caractérisation de catalyseurs de 3 mm à 6 mm est basée sur l'utilisation d'un tamis de 425 $\mu$m qui va permettre notamment de séparer les fines créées lors de l'écrasement. L'utilisation d'un tamis de 425 $\mu$m reste adaptée pour des particules de diamètre supérieur à 1,6 mm, mais doit être adaptée selon la granulométrie des agglomérés que l'on cherche à caractériser.

Les adsorbants du procédé selon la présente invention, généralement sous forme de billes ou d'extrudés, ont en général un diamètre moyen en volume ou une longueur, i.e. plus grande dimension dans le cas des agglomérés non sphériques, comprise entre 0,2 mm et 2 mm, et en particulier comprise entre 0,2 mm et 0,8 mm et de préférence entre 0,4 mm et 0,65 mm. Par conséquent, un tamis de 100 $\mu$m est utilisé à la place du tamis de 425 $\mu$m mentionné dans la méthode Shell standard SMS1471-74.

Le protocole de mesure est le suivant : un échantillon de 20 cm$^3$ d'adsorbants, préalablement tamisé avec le tamis adapté (100 $\mu$m) et préalablement séché à l'étuve pendant au moins 2 heures à 250°C (au lieu de 300°C mentionné dans la méthode Shell standard SMS1471-74), est placé dans un cylindre métallique de section interne connue. Une force croissante est imposée par paliers sur cet échantillon par l'intermédiaire d'un piston, à travers un lit de 5 cm$^3$ de billes d'acier afin de mieux répartir la force exercée par le piston sur les agglomérés (utilisation de billes de 2 mm de diamètre pour des particules de forme sphérique de diamètre strictement inférieur à 1,6 mm). Les fines obtenues aux différents paliers de pression sont séparées par tamisage (tamis adapté de 100 $\mu$m) et pesées.

La résistance à l'écrasement en lit est déterminée par la pression en mégaPascal (MPa) pour laquelle la quantité de fines cumulées passant à travers le tamis s'élève à 0,5% pondéral de l'échantillon. Cette valeur est obtenue en traçant sur un graphique la masse de fines obtenue en fonction de la force appliquée sur le lit d'adsorbant et en interpolant à 0,5 % massique de fines cumulées. La résistance mécanique à l'écrasement en lit est typiquement comprise entre quelques centaines de kPa et quelques dizaines de MPa et généralement comprise entre 0,3 MPa et 3,2 MPa. La précision est de manière classique inférieure à 0,1 MPa.

**Quantité massique des fractions zéolithiques des adsorbants zéolithiques**

La quantité massique des fractions zéolithiques est mesurée par analyse par diffraction de rayons X, connue de l'homme du métier sous l'acronyme DRX. Cette analyse est réalisée sur un appareil de la marque Bruker, puis la quantité des fractions zéolithiques est évaluée au moyen du logiciel TOPAS de la société Bruker.

**Volume microporeux, surface externe et diamètre des mésopores**

La cristallinité des agglomérés est également évaluée par mesure de leur volume microporeux en le comparant à celui d'une référence appropriée (zéolithe 100% cristalline dans des conditions de traitements cationiques identiques ou zéolithe théorique). Ce volume microporeux est déterminé à partir de la mesure de l'isotherme d'adsorption de gaz, tel que l'azote, à sa température de liquéfaction.

Préalablement à l'adsorption, l'adsorbant zéolithique est dégazé entre 300°C et 450°C pendant une durée comprise entre 9 heures et 16 heures, sous vide (P < 6,7.10$^{-4}$ Pa). La mesure de l'isotherme d'adsorption d'azote à 77K est ensuite effectuée sur un appareil de type ASAP 2020 M de Micromeritics, en prenant au moins 35 points de mesure à des pressions relatives de rapport $P/P_0$ compris entre 0,002 et 1.

Le volume microporeux ainsi que la surface externe sont déterminés à partir de l'isotherme obtenue, par la méthode du t-plot en appliquant la norme ISO 15901-3:2007 et en calculant l'épaisseur statistique t par l'équation de Harkins-Jura. Le volume microporeux et la surface externe sont obtenus par régression linéaire sur les points du t-plot compris entre 0,45 nm et 0,57 nm, respectivement à partir de l'ordonnée à l'origine et de la pente de la régression linéaire. Le volume microporeux évalué s'exprime en cm$^3$ d'adsorbat liquide par gramme d'adsorbant anhydre. La surface externe s'exprime en m$^2$ par gramme d'adsorbant anhydre.

L'interprétation de l'isotherme d'adsorption d'azote à 77K par la méthode de Barrett-Joyner-Halenda (méthode BJH, proposée en 1951) permet en outre d'obtenir la distribution de taille de pores, et notamment la distribution des mésopores. La distribution en volume de taille de mésopores est représentée par la courbe $d$V/$d$Dm en fonction du diamètre moyen des pores $Dm.$

La largeur à mi-hauteur de la distribution en volume $d$V/$d$Dm est donnée par la différence entre les deux diamètres moyens pour lesquels la valeur $d$V/$d$Dm serait égale à la moitié de sa valeur maximale $f_{max}$, au sommet du pic. Ces deux diamètres moyens sont obtenus par interpolation entre les points recherchés de part et d'autre du mode, pour lesquels $d$V/$d$Dm encadre la valeur $f_{max}$/2. Cette largeur à mi-hauteur ou FWHM (acronyme anglais pour « Full width at half maximum ») d'une distribution f(x) de valeur maximale est $f_{max}$ est schématiquement illustrée à titre d'exemple à la Figure 1.

### Volume macroporeux et mésoporeux

Les volumes macroporeux et mésoporeux sont mesurés par porosimétrie par intrusion de mercure. Un porosimètre à mercure type Autopore® 9500 de Micromeritics est utilisé pour analyser la répartition du volume poreux contenu dans les macropores et dans les mésopores.

La méthode expérimentale, décrite dans le manuel opératoire de l'appareil faisant référence à la norme ASTM D4284-83, consiste à placer un échantillon d'adsorbant (matériau granulaire zéolithique à mesurer) (de perte au feu connue) préalablement pesé, dans une cellule du porosimètre, puis, après un dégazage préalable (pression d'évacuation de 30 $\mu$m Hg pendant au moins 10 min), à remplir la cellule avec du mercure à une pression donnée (0,0036 MPa), et ensuite à appliquer une pression croissante par palier jusqu'à 400 MPa afin de faire pénétrer progressivement le mercure dans le réseau poreux de l'échantillon.

La relation entre la pression appliquée et le diamètre apparent des pores est établie en supposant des pores cylindriques, un angle de contact entre le mercure et la paroi des pores de 140° et une tension de surface du mercure de 485 dynes/cm. La quantité cumulée de mercure introduite en fonction de la pression appliquée est enregistrée. On fixe à 0,2 MPa la valeur à partir de laquelle le mercure remplit tous les vides inter-granulaires, et on considère qu'au delà le mercure pénètre dans les pores du matériau granulaire. Le volume de grain (Vg) est alors calculé en soustrayant le volume cumulé de mercure à cette pression (0,2 MPa) au volume de la cellule du porosimètre, et en divisant cette différence par la masse du matériau granulaire équivalent anhydre, c'est-à-dire la masse dudit matériau corrigée de la perte au feu.

Le volume macroporeux du matériau granulaire est défini comme étant le volume cumulé de mercure introduit à une pression comprise entre 0,2 MPa et 30 MPa, correspondant au volume contenu dans les pores de diamètre apparent supérieur à 50 nm. Le volume mésoporeux du matériau granulaire est défini comme étant le volume cumulé de mercure introduit à une pression comprise entre 30 MPa et 400 MPa.

Dans le présent document, les volumes macroporeux et mésoporeux des adsorbants zéolithiques, exprimés en $cm^3.g^{-1}$, sont ainsi mesurés par intrusion de mercure et rapportés à la masse de l'échantillon en équivalent anhydre, c'est-à-dire la masse dudit matériau corrigée de la perte au feu.

### Perte au feu des adsorbants zéolithiques

La perte au feu est déterminée en atmosphère oxydante, par calcination de l'échantillon à l'air à une température de 950°C $\pm$ 25°C, comme décrit dans la norme NF EN 196-2 (avril 2006). L'écart type de mesure est inférieur à 0,1%.

### Caractérisation de l'adsorption en phase liquide par perçage

La technique utilisée pour caractériser l'adsorption de molécules en phase liquide sur un solide poreux est la technique dite de perçage, décrite par Ruthven dans « Principles of Adsorption and Adsorption Processes » (John Wiley & Sons, (1984), Chapitres 8 et 9) qui définit la technique des courbes de perçage (« breakthrough curves ») comme l'étude de la réponse à l'injection d'un échelon de constituants adsorbables.

L'analyse du temps moyen de sortie (premier moment) des courbes de perçage fournit une information sur les quantités adsorbées et permet également d'évaluer les sélectivités, c'est-à-dire le facteur de séparation, entre deux constituants adsorbables. L'injection d'un constituant non adsorbable utilisé comme traceur est conseillée pour l'estimation des volumes non-sélectifs. L'analyse de la dispersion (second moment) des courbes de perçage, permet d'évaluer la hauteur équivalente de plateaux théoriques, basée sur la représentation d'une colonne par un nombre fini de réacteurs hypothétiques idéalement agités (étages théoriques), qui est une mesure directe de la dispersion axiale et de la résistance au transfert de matière du système.

**Exemple 1** : Synthèse de zéolithe Y à haute surface externe.

La zéolithe Y à haute surface externe est synthétisée de manière directe.

*Étape 1) : Préparation du gel de croissance dans réacteur agité.*

Dans un réacteur en inox de 3 litres muni d'une double enveloppe chauffante, d'une sonde température et d'un agitateur, on prépare un gel de croissance en mélangeant une solution d'aluminate contenant 1146 g de sol de silice Ludox AM à 30 % de silice dans une solution d'aluminate contenant 198 g d'hydroxyde de sodium (NaOH), 138 g

d'alumine trihydratée (Al$_2$O$_3$, 3H$_2$O, contenant 65,2% en poids d'Al$_2$O$_3$) et 800 g d'eau à 25°C en 25 minutes avec une vitesse d'agitation de 300 tr.min$^{-1}$.

La stoechiométrie du gel de croissance est la suivante : 2,75 Na$_2$O/Al$_2$O$_3$/8.20 SiO$_2$ / 120 H$_2$O. L'homogénéisation du gel de croissance est réalisée sous agitation à 300 tr.min$^{-1}$, pendant 40 minutes à 25°C.

### Étape 2) : Introduction dans le milieu réactionnel du gel de nucléation

On introduit dans le milieu réactionnel 153 g d'un gel de nucléation (12 Na$_2$O, Al$_2$O$_3$, 10 SiO$_2$, 180 H$_2$O mûri pendant 1 heure à 40°C), après 5 minutes d'homogénéisation à 300 tr.min$^{-1}$ on baisse la vitesse d'agitation à 100 tr.min$^{-1}$ pendant 30 minutes.

### Étape 3) : Introduction de l'agent structurant

On ajoute 87,5 g de solution de TPOAC à 60% dans le méthanol avec une vitesse d'agitation de 300 tr.min$^{-1}$ (ratio molaire TPOAC/Al$_2$O$_3$ = 0,12). Après 1 h d'homogénéisation, on baisse la vitesse d'agitation à 100 tr.min$^{-1}$.

### Étape 4) : Phase de mûrissement

On maintient le milieu réactionnel agité à 100 tr.min$^{-1}$ à 25°C, pendant 17 heures, puis on démarre la cristallisation.

### Étape 5) : Cristallisation

On maintient la vitesse d'agitation à 100 tr.min$^{-1}$, et on fixe la consigne de la double enveloppe du réacteur à 108°C afin que le milieu réactionnel monte en température à 98°C en 80 minutes. Après 28 heures de palier à 98°C, on refroidit le milieu réactionnel en faisant circuler de l'eau froide dans la double enveloppe pour stopper la cristallisation.

### Étape 6) : Filtration / lavage

Les solides sont récupérés sur fritté puis lavés avec de l'eau permutée jusqu'à pH neutre.

### Étape 7) : Séchage / Calcination

Afin de caractériser le produit, le séchage est réalisé en étuve à 90°C pendant 8 heures, la perte au feu du produit séché est de 22 % en poids.

La calcination du produit séché nécessaire pour libérer à la fois la microporosité (eau) et la mésoporosité en éliminant l'agent structurant est effectuée avec le profil de température suivant : 30 minutes de montée à 200°C, puis 1 heure de palier à 200°C, puis 3 heures de montée à 550°C, et enfin 1,5 heures de palier à 550°C.

On obtient des cristaux de zéolithe Y pure (identification par spectre de Diffraction des Rayons X), à haute surface externe de rapport atomique Si/Al de 2,30. Le volume microporeux et la surface externe, mesurés selon la méthode du t-plot à partir de l'isotherme d'adsorption d'azote à 77 °K après dégazage sous vide à 400°C pendant 10 heures, sont respectivement 0,214 cm$^3$.g$^{-1}$ et 115 m$^2$.g$^{-1}$. Le diamètre moyen en nombre des cristaux est de 1,3 $\mu$m.

### Exemple 2 : Préparation d'adsorbant de zéolithe Y mésoporeuse

Dans ce qui suit, une masse exprimée en équivalent anhydre signifie une masse de produit diminuée de sa perte au feu.

On prépare un mélange homogène constitué de 16 g de cristaux de zéolithe Y à haute surface externe obtenue à l'exemple 1, de 4 g de kaolin, ainsi que de la quantité d'eau qui permet l'extrusion du mélange. La perte au feu de la pâte avant extrusion est de 40%.

On forme des extrudés de 1,6 mm de diamètre. Les extrudés sont séchés une nuit en étuve ventilée à 80°C. Ils sont ensuite calcinés pendant 2 heures à 550°C sous balayage à l'azote, puis 2 heures à 550°C sous balayage à l'air sec décarbonaté.

Les extrudés sont ensuite concassés de manière à récupérer des grains dont le diamètre équivalent est égal à 0,6 mm. La résistance mécanique à l'écrasement en lit des grains obtenus ci-dessus est de 2,2 MPa.

Le volume microporeux et la surface externe, mesurés selon la méthode du t-plot à partir de l'isotherme d'adsorption d'azote à 77 °K après dégazage sous vide à 400°C pendant 10 heures, sont respectivement 0,170 cm$^3$.g$^{-1}$ et 137 m$^2$.g$^{-1}$. L'analyse par diffraction des rayons X confirme la présence d'une seule phase zéolithique FAU de type Y.

<u>Exemple 3 :</u> (comparatif) **Préparation d'adsorbant de zéolithe Y non mésoporeuse (conventionnelle)**

À titre de comparaison pour la préparation des agglomérés, on utilise une zéolithe Y pure commerciale, la CBV100 commercialisée par la société Zeolyst International, non mésoporeuse de rapport atomique Si/Al égal à 2,6, un diamètre moyen en nombre de 0,6 $\mu$m, de volume microporeux et de surface externe mesurés selon la méthode du t-plot à partir de l'isotherme d'adsorption d'azote à 77 °K après dégazage sous vide à 400°C pendant 10 heures, qui sont respectivement égaux à 0,345 $cm^3.g^{-1}$ et 23 $m^2.g^{-1}$.

Les opérations de l'exemple 2 sont répétées à l'identique en substituant la zéolithe Y mésoporeuse par la zéolithe Y non mésoporeuse de référence (CBV 100 de Zeolyst).

Le volume microporeux et la surface externe, mesurés selon la méthode du t-plot à partir de l'isotherme d'adsorption d'azote à 77 °K après dégazage sous vide à 400°C pendant 10 heures, sont respectivement 0,278 $cm^3.g^{-1}$ et 42 $m^2.g^{-1}$.

L'observation au microscope électronique à transmission MET (Figures 2a et 2b, MET des agglomérés selon l'exemple 2) et Figures 3a et 3b des agglomérés conventionnels préparés dans l'exemple 3) révèle la présence des cavités de taille nanométrique (mésopores) dans les agglomérés selon l'invention Exemple 2 (Figures 2a et 2b).

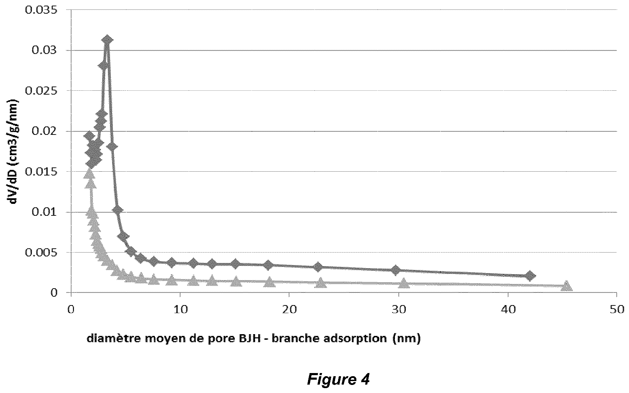

La comparaison des distributions des tailles de pores déterminées par la méthode BJH, à partir de l'isotherme d'adsorption d'azote à 77K et illustrée par la Figure 4 qui montre bien que les agglomérés selon l'invention présentent une distribution de tailles de pores unimodale et étroite dans le domaine des mésopores (c'est-à-dire pores de diamètre moyen compris entre 2 nm et 50 nm) centrée sur 3,4 nm et de largeur à mi-hauteur de 2 nm (cf. courbe matérialisée par des losanges foncés). Les agglomérés conventionnels de zéolithe Y non mésoporeuse (cf. courbe matérialisée par des triangles clairs) ne présentent pas de distribution unimodale dans le domaine des mésopores (c'est-à-dire pores de diamètre moyen compris entre 2 nm et 50 nm), en effet aucun pic présentant un maximum n'est observé sur la distribution de tailles de pores.

Le Tableau 1 ci-dessous présente les caractéristiques des adsorbants préparés aux Exemple 2 et 3.

**-- Tableau 1 --**

| | Adsorbant de l'Exemple 2 (selon l'invention) | Adsorbant de l'Exemple 3 (conventionnel) |
|---|---|---|
| **Volume microporeux par t-plot ($cm^3.g^{-1}$)** | 0,170 | 0,278 |
| **Surface externe ($m^2.g^{-1}$)** | 137 | 42 |
| **Volume mésoporeux ($cm^3.g^{-1}$)** | 0,268 | 0,111 |
| **Diamètre mésopore - Mode (nm)** | 3,4 | indéfini |
| **dV/dD mode ($cm^3.g^{-1}.nm^{-1}$)** | 0,031 | indéfini |
| **Largeur à mi-hauteur (nm)** | 2,0 | indéfini |

**Exemple 4 : Test de perçage sur adsorbants des exemples 2 et 3**

Un test de perçage (chromatographie frontale) est ensuite réalisé sur ces adsorbants pour évaluer leur efficacité. La quantité d'adsorbant utilisée pour ce test est d'environ 26 g.

Le mode opératoire pour obtenir les courbes de perçage est le suivant :

- Remplissage de la colonne par le tamis et mise en place dans le banc de test.
- Remplissage par le solvant à température ambiante.
- Montée progressive à la température d'adsorption sous flux de solvant (5 $cm^3.min^{-1}$).
- Injection de solvant à 30 $cm^3.min^{-1}$ lorsque la température d'adsorption est atteinte.
- Permutation solvant/charge pour injecter la charge (30 $cm^3.min^{-1}$).
- L'injection de la charge est ensuite maintenue un temps suffisant pour atteindre l'équilibre thermodynamique (c'est-à-dire jusqu'à ce que la concentration en solvant dans l'effluent soit nulle).
- Collecte et analyse de l'effluent du perçage.

Le solvant utilisé est le para-diéthylbenzène. La composition de la charge est la suivante :

- Métaxylène : 45% poids

- Orthoxylène : 45% poids

- Iso-octane : 10% poids (celui-ci est utilisé comme traceur pour l'estimation des volumes non-sélectifs et n'intervient pas dans la séparation)

Le test est réalisé avec une température d'adsorption de 140°C. La pression est suffisante pour que la charge reste en phase liquide, soit 1 MPa. La vitesse superficielle (débit/section de la colonne) de circulation du liquide à la température du test est d'environ 1,2 cm.s$^{-1}$ pour l'ensemble des tests.

La sélectivité du méta-xylène (MX) par rapport à l'ortho-xylène (OX) ($\alpha_{MX/OX}$) est calculée à partir des volumes adsorbés $q_{MX}$ et $q_{OX}$ des composés MX et OX (ces derniers étant déterminés par bilan matière à partir de l'analyse de l'effluent du perçage) et de la composition de la charge (charge dans laquelle la fraction volumique des composés est

$$y_{MX} \text{ et } y_{OX}) : \alpha_{MX/OX} = \frac{q_{MX}}{q_{OX}} \frac{y_{OX}}{y_{MX}} \ .$$

Les résultats du perçage sont consignés dans le Tableau 2 ci-dessous :

-- Tableau 2 --

| Adsorbant | sélectivité MX/OX | Capacité d'adsorption (%) | HEPT MX (%) |
|---|---|---|---|
| de l'exemple 2 (invention) | 1,70 | 11,3 | 6,7 |
| de l'exemple 3 (comparatif) | 1,83 | 13,4 | 10,0 |

Légende

- Capacité d'adsorption exprimée en % (cm$^3$ de C$_8$-aromatiques adsorbés par cm$^3$ de colonne)
- HEPT = Hauteur Équivalente de Plateaux Théoriques mesurée sur le méta-xylène exprimée en % de longueur de colonne
- MX = Méta-Xylène ; OX = Ortho-Xylène

On observe que l'utilisation de l'adsorbant selon l'invention permet de réduire considérablement la Hauteur Équivalente de Plateaux Théoriques mesurée pour le méta-xylène, indiquant une amélioration du transfert de matière.

**Revendications**

1. Procédé de séparation du *méta*-xylène mettant en oeuvre un adsorbant zéolithique comprenant au moins une zéolithe faujasite de type Y comprenant du sodium ou du sodium et du lithium, procédé dans lequel la surface externe dudit adsorbant zéolithique, mesurée par adsorption d'azote, est supérieure à 40 m$^2$.g$^{-1}$, de préférence supérieure à 50 m$^2$.g$^{-1}$, et de préférence encore comprise entre 60 m$^2$.g$^{-1}$ et 200 m$^2$.g$^{-1}$, et plus préférentiellement comprise entre 80 m$^2$.g$^{-1}$ et 200 m$^2$.g$^{-1}$, et de manière encore plus préférée entre 100 m$^2$.g$^{-1}$ et 200 m$^2$.g$^{-1}$, ladite surface externe étant associée à une population de mésopores de diamètre moyen compris entre 2 nm et 50 nm.

2. Procédé selon la revendication 1, dans lequel la distribution de taille de pores dudit adsorbant zéolithique, telle que déterminée par la méthode Barrett-Joyner-Halenda à partir de l'isotherme d'adsorption d'azote à 77K, pour les pores compris entre 2 nm et 50 nm, est unimodale et étroite.

3. Procédé selon la revendication 1 ou la revendication 2, dans lequel, l'adsorbant zéolithique est un adsorbant à base de zéolithe(s) FAU de type Y, le rapport atomique Si/Al dudit adsorbant étant supérieur à 1,50, avantageusement compris entre 1,50 et 6,50, de préférence entre 2,00 et 6,00, de préférence encore entre 2,00 et 4,00, et de manière encore plus préférée entre 2,20 et 2,80.

4. Procédé selon l'une quelconque des revendications précédentes, dans lequel ledit adsorbant zéolithique présente une fraction massique de zéolithe(s) FAU de type Y supérieure ou égale à 80% par rapport au poids total de l'adsorbant, le complément à 100% étant de préférence constitué de phase non zéolithique.

**5.** Procédé selon l'une quelconque des revendications précédentes, dans lequel ledit adsorbant zéolithique présente une teneur en lithium inférieure à 8%, de préférence comprise entre 0 et 4%, exprimée en poids d'oxyde de lithium $Li_2O$ par rapport à la masse totale de l'adsorbant.

**6.** Procédé selon l'une quelconque des revendications précédentes, dans lequel l'adsorbant zéolithique comprend à la fois des pores dont le diamètre est supérieur à 50 nm, de préférence compris entre 50 nm et 400 nm, des pores dont le diamètre est compris entre 2 nm et 50 nm, bornes non incluses, et des pores dont le diamètre est inférieur à 2 nm.

**7.** Procédé selon l'une quelconque des revendications précédentes, dans lequel l'adsorbant zéolithique présente un volume total contenu dans les macropores et les mésopores (somme du volume macroporeux et du volume mésoporeux) mesuré par intrusion de mercure, compris entre 0,20 $cm^3.g^{-1}$ et 0,70 $cm^3.g^{-1}$, de préférence compris entre 0,20 $cm^3.g^{-1}$ et 0,55 $cm^3.g^{-1}$ et de manière très préférée compris entre 0,20 $cm^3.g^{-1}$ et 0,50 $cm^3.g^{-1}$.

**8.** Procédé selon l'une quelconque des revendications précédentes, dans lequel l'adsorbant zéolithique présente un ratio (volume macroporeux)/(volume macroporeux + volume mésoporeux) compris entre 0,2 et 1, de manière préférée compris entre 0,4 et 0,8, et de manière encore plus préférée entre 0,45 et 0,65.

**9.** Procédé selon l'une quelconque des revendications précédentes, dans lequel l'adsorbant zéolithique mis en oeuvre présente un volume microporeux évalué par la méthode de t-plot à partir de l'isotherme d'adsorption d'azote ($N_2$) à une température de 77 K, compris entre 0,145 $cm^3.g^{-1}$ et 0,350 $cm^3.g^{-1}$, mieux entre 0,155 $cm^3.g^{-1}$ et 0,350 $cm^3.g^{-1}$, de préférence compris entre 0,165 $cm^3.g^{-1}$ et 0,350 $cm^3.g^{-1}$, mieux encore entre 0,175 $cm^3.g^{-1}$ et 0,350 $cm^3.g^{-1}$, et de préférence encore compris entre 0,205 $cm^3.g^{-1}$ et 0,320 $cm^3.g^{-1}$.

**10.** Procédé selon l'une quelconque des revendications précédentes, dans lequel l'adsorbant zéolithique comprend des cristaux mésoporeux d'au moins une zéolithe Y, lesdits cristaux présentant un diamètre moyen en nombre compris entre 0,1 $\mu$m et 20 $\mu$m, de préférence entre 0,5 $\mu$m et 20 $\mu$m, de préférence encore entre 0,7 $\mu$m et 10 $\mu$m, et plus préférentiellement encore entre 0,8 $\mu$m et 10 $\mu$m, et plus préférentiellement encore entre 1 $\mu$m et 5 $\mu$m.

**11.** Procédé selon l'une quelconque des revendications précédentes, dans lequel les cristaux de zéolithe(s) dudit adsorbant zéolithique sont préparés par synthèse directe mettant en oeuvre un ou plusieurs agents structurants ou gabarits sacrificiels.

**12.** Procédé selon l'une quelconque des revendications précédentes, dans lequel le produit désiré (*méta*-xylène) est séparé par chromatographie liquide d'adsorption préparative (en batch), et avantageusement en continu en lit mobile simulé, c'est-à-dire à contre-courant simulé ou à co-courant simulé, et plus particulièrement à contre-courant simulé.

## Patentansprüche

**1.** Verfahren zur Trennung von meta-Xylol unter Verwendung eines zeolithischen Adsorptionsmittels, umfassend mindestens einen Faujasit-Zeolith vom Typ Y, umfassend Natrium oder Natrium und Lithium, Verfahren, wobei die durch Stickstoffadsorption gemessene externe Oberfläche des zeolithischen Adsorptionsmittels größer als 40 $m^2.g^{-1}$, vorzugsweise größer als 50 $m^2.g^{-1}$ ist und noch bevorzugter zwischen 60 $m^2.g^{-1}$ und 200 $m^2.g^{-1}$ und noch bevorzugter zwischen 80 $m^2.^{-1}$ und 200 $m^2.g^{-1}$ und noch bevorzugter zwischen 100 $m^2.g^{-1}$ und 200 $m^2.g^{-1}$ beträgt, wobei die externe Oberfläche mit einer Population von Mesoporen mit einem mittleren Durchmesser zwischen 2 nm und 50 nm verbunden ist.

**2.** Verfahren nach Anspruch 1, wobei die Porengrößenverteilung des zeolithischen Adsorptionsmittels, wie durch das Verfahren nach Barrett, Joyner und Halenda ausgehend von der Stickstoff-Adsorptionsisotherme bei 77 K bestimmt, für die Poren zwischen 2 nm und 50 nm unimodal und eng ist.

**3.** Verfahren nach Anspruch 1 oder 2, wobei das zeolithische Adsorptionsmittel ein Adsorptionsmittel auf der Basis von FAU-Zeolith (en) vom Typ Y ist, wobei das Si/Al-Atomverhältnis des Adsorptionsmittels größer als 1,50 ist, vorteilhafterweise zwischen 1,50 und 6,50, vorzugsweise zwischen 2,00 und 6,00, noch bevorzugter zwischen 2,00 und 4,00 und insbesondere zwischen 2,20 und 2,80 beträgt.

**4.** Verfahren nach einem der vorhergehenden Ansprüche, wobei das zeolithische Adsorptionsmittel einen Massenanteil an FAU-Zeolith (en) vom Typ Y aufweist, der größer oder gleich 80 %, bezogen auf das Gesamtgewicht des Adsorptionsmittels, ist, wobei die Ergänzung zu 100 % vorzugsweise aus nicht-zeolithischer Phase besteht.

5. Verfahren nach einem der vorhergehenden Ansprüche, wobei das zeolithische Adsorptionsmittel einen Lithiumgehalt von weniger als 8 Gew.%, vorzugsweise von zwischen 0 und 4 Gew.% Lithiumoxid $Li_2O$, bezogen auf die Gesamtmasse des Adsorptionsmittels, aufweist.

6. Verfahren nach einem der vorhergehenden Ansprüche, wobei das zeolithische Adsorptionsmittel sowohl Poren, deren Durchmesser größer als 50 nm ist, vorzugsweise zwischen 50 nm und 400 nm beträgt, als auch Poren, deren Durchmesser zwischen 2 nm und 50 nm, Grenzwerte ausgeschlossen, beträgt, und Poren aufweist, deren Durchmesser weniger als 2 nm beträgt.

7. Verfahren nach einem der vorhergehenden Ansprüche, wobei das zeolithische Adsorptionsmittel ein Gesamtvolumen, das in den Makroporen und Mesoporen enthalten ist (Summe des makroporösen Volumens und des mesoporösen Volumens), gemessen durch Quecksilberintrusion, zwischen 0,20 $cm^3.g^{-1}$ und 0,70 $cm^3.g^{-1}$, vorzugsweise zwischen 0,20 $cm^3.g^{-1}$ und 0,55 $cm^3.g^{-1}$ und insbesondere zwischen 0,20 $cm^3.g^{-1}$ und 0,50 $cm^3.g^{-1}$ aufweist.

8. Verfahren nach einem der vorhergehenden Ansprüche, wobei das zeolithische Adsorptionsmittel ein Verhältnis (makroporöses Volumen)/(makroporöses Volumen + mesoporöses Volumen) zwischen 0,2 und 1, vorzugsweise zwischen 0,4 und 0,8 und insbesondere zwischen 0,45 und 0,65 aufweist.

9. Verfahren nach einem der vorhergehenden Ansprüche, wobei das eingesetzte zeolithische Adsorptionsmittel ein mikroporöses Volumen, das nach der t-plot Methode ausgehend von der Stickstoff-adsorptionsisotherme ($N_2$) bei einer Temperatur von 77 K bewertet wird, zwischen 0,145 $cm^3.g^{-1}$ und 0,350 $cm^3.g^{-1}$, besser zwischen 0,155 $cm^3.g^{-1}$ und 0,350 $cm^3.g^{-1}$, vorzugsweise zwischen 0,165 $cm^3.g^{-1}$ und 0,350 $cm^3.g^{-1}$, noch bevorzugter zwischen 0,175 $cm^3.g^{-1}$ und 0,350 $cm^3.g^{-1}$ und insbesondere zwischen 0,205 $cm^3.g^{-1}$ und 0,320 $cm^3.g^{-1}$ aufweist.

10. Verfahren nach einem der vorhergehenden Ansprüche, wobei das zeolithische Adsorptionsmittel mesoporöse Kristalle von mindestens einem Y-Zeolithen umfasst, wobei die Kristalle einen zahlenmittleren Durchmesser zwischen 0,1 μm und 20 μm, vorzugsweise zwischen 0,5 μm und 20 μm, bevorzugter zwischen 0,7 μm und 10 μm und noch bevorzugter zwischen 0,8 μm und 10 μm und insbesondere zwischen 1 μm und 5 μm aufweisen.

11. Verfahren nach einem der vorhergehenden Ansprüche, wobei die Zeolithkristalle des zeolithischen Adsorptionsmittels durch direkte Synthese unter Verwendung von einem oder mehreren Strukturierungsmitteln oder Opfer-Schablonen hergestellt werden.

12. Verfahren nach einem der vorhergehenden Ansprüche, wobei das gewünschte Produkt (meta-Xylol) durch präparative Adsorptionsflüssigkeitschromatographie (chargenweise) und vorteilhafterweise kontinuierlich im simulierten Bewegtbett, d.h. mit simuliertem Gegenstrom oder simuliertem Gleichstrom, und insbesondere mit simuliertem Gegenstrom, getrennt wird.


**Claims**

1. Process for separating meta-xylene using a zeolite adsorbent comprising at least one faujasite-type zeolite Y comprising sodium or sodium and lithium, in which process the external surface area of said zeolite adsorbent, measured by nitrogen adsorption, is greater than 40 $m^2.g^{-1}$, preferably greater than 50 $m^2.g^{-1}$, and more preferably between 60 $m^2.g^{-1}$ and 200 $m^2.g^{-1}$, and more preferentially between 80 $m^2.g^{-1}$ and 200 $m^2.g^{-1}$, and even more preferably between 100 $m^2.g^{-1}$ and 200 $m^2.g^{-1}$, said external surface area being associated with a population of mesopores with a mean diameter of between 2 nm and 50 nm.

2. Process according to Claim 1, in which the pore size distribution of said zeolite adsorbent, as determined via the Barrett-Joyner-Halenda method from the nitrogen adsorption isotherm at 77 K, for pores between 2 nm and 50 nm, is unimodal and narrow.

3. Process according to Claim 1 or Claim 2, in which the zeolite adsorbent is an adsorbent based on FAU-type zeolite(s) Y, the Si/Al atomic ratio of said adsorbent being greater than 1.50, advantageously between 1.50 and 6.50, preferably between 2.00 and 6.00, more preferably between 2.00 and 4.00 and even more preferably between 2.20 and 2.80.

4. Process according to any one of the preceding claims, in which said zeolite adsorbent has a mass fraction of FAU-type zeolite(s) Y of greater than or equal to 80% relative to the total weight of the adsorbent, the remainder to 100% preferably being formed from non-zeolite phase.

5. Process according to any one of the preceding claims, in which said zeolite adsorbent has a lithium content of less than 8%, preferably between 0 and 4%, expressed as weight of lithium oxide $Li_2O$ relative to the total mass of the adsorbent.

6. Process according to any one of the preceding claims, in which the zeolite adsorbent simultaneously comprises pores whose diameter is greater than 50 nm, preferably between 50 nm and 400 nm, pores whose diameter is between 2 nm and 50 nm, limits not included, and pores whose diameter is less than 2 nm.

7. Process according to any one of the preceding claims, in which the zeolite adsorbent has a total volume contained in the macropores and the mesopores (sum of the macropore volume and of the mesopore volume) measured by mercury intrusion, of between 0.20 $cm^3.g^{-1}$ and 0.70 $cm^3.g^{-1}$, preferably between 0.20 $cm^3.g^{-1}$ and 0.55 $cm^3.g^{-1}$ and very preferably between 0.20 $cm^3.g^{-1}$ and 0.50 $cm^3.g^{-1}$.

8. Process according to any one of the preceding claims, in which the zeolite adsorbent has a ratio (macropore volume)/(macropore volume + mesopore volume) of between 0.2 and 1, preferably between 0.4 and 0.8 and even more preferably between 0.45 and 0.65.

9. Process according to any one of the preceding claims, in which the zeolite adsorbent used has a micropore volume, evaluated by the t-plot method from the nitrogen ($N_2$) adsorption isotherm at a temperature of 77 K, of between 0.145 $cm^3.g^{-1}$ and 0.350 $cm^3.g^{-1}$, better still between 0.155 $cm^3.g^{-1}$ and 0.350 $cm^3.g^{-1}$, preferably between 0.165 $cm^3.g^{-1}$ and 0.350 $cm^3.g^{-1}$, better still between 0.175 $cm^3.g^{-1}$ and 0.350 $cm^3.g^{-1}$, and more preferably between 0.205 $cm^3.g^{-1}$ and 0.320 $cm^3.g^{-1}$.

10. Process according to any one of the preceding claims, in which the zeolite adsorbent comprises mesoporous crystals of at least one zeolite Y, said crystals having a number-mean diameter of between 0.1 pm and 20 $\mu$m, preferably between 0.5 $\mu$m and 20 $\mu$m, more preferably between 0.7 $\mu$m and 10 $\mu$m, even more preferentially between 0.8 $\mu$m and 10 $\mu$m and even more preferentially between 1 $\mu$m and 5 $\mu$m.

11. Process according to any one of the preceding claims, in which the zeolite crystals of said zeolite adsorbent are prepared by direct synthesis using one or more structuring agents or sacrificial templates.

12. Process according to any one of the preceding claims, in which the desired product (meta-xylene) is separated by preparative adsorption liquid chromatography (batchwise), and advantageously continuously in a simulated moving bed, i.e. in simulated counter-current mode or in simulated cocurrent mode, and more particularly in simulated counter-current mode.

Figure 1

Figure 2a

Figure 2b

Figure 3a

Figure 3b

**Figure 4**

## RÉFÉRENCES CITÉES DANS LA DESCRIPTION

**Documents brevets cités dans la description**

- US 4306107 A
- US 4326092 A
- US 5382747 A
- US 5900523 A
- US 7728187 B
- FR 2889698
- FR 2889699
- US 2985589 A
- US 7785563 B
- CN 103214003
- WO 2013106816 A
- WO 2007043731 A

**Littérature non-brevet citée dans la description**

- **RUTHVEN.** « Principles of Adsorption and Adsorption Processes ». John Wiley & Sons, 1984, 326, , 407
- PRINCIPLES OF ADSORPTION AND ADSORPTION PROCESSES. 243
- **E. P. BARRETT ; L. G. JOYNER ; P. P. HALENDA.** The Détermination of Pore Volume and Area Distributions in Porous Substances. I. Computations form Nitrogen Isotherms. *J. Am. Chem. Soc.,* 1951, vol. 73 (1), 373-380
- **D. VERBOEKEND.** *Adv. Funct. Mater.,* 2012, vol. 22, 916-928
- **RUTHVEN.** Principles of Adsorption and Adsorption Processes. John Wiley & Sons, 1984